# EUROPEAN PATENT APPLICATION

(11) **EP 2 335 570 A1**
(43) Date of publication of application: **22.06.2011**
(21) Application number: 09813112.1
(22) Date of filing: 10.09.2009
(51) Int. Cl.: A61B 5/0408, A61B 5/0478, A61B 5/0492, A61F 2/60, A61F 2/72

(54) **WEARING TOOL FOR MEASURING BIOLOGICAL SIGNAL, AND WEARING-TYPE MOTION ASSISTING DEVICE**

(30) Priority: 10.09.2008 JP 2008232091
(71) Applicant: University of Tsukuba, Tsukuba-shi, Ibaraki 305-8577 (JP)
(72) Inventor: SANKAI Yoshiyuki, Tsukuba-Shi Ibaraki 305-8577 (JP)
(74) Representative: Ilgart, Jean-Christophe
(86) International application number: PCT/JP2009/065825
(87) International publication number: WO 2010/029966

(57) **Abstract**

A biological signal measuring wearing device configured to measure a biological signal from the body surface of a wearer includes a wearing device body configured to cover the body surface of the wearer, a biological signal detecting part provided on a predetermined part of the inner surface of the wearing device body and configured to detect the biological signal from the body surface of the wearer, and a signal communicating part configured to output the signal detected by the biological signal detecting part.

## Description

### TECHNICAL FIELD

The present invention relates to biological signal measuring wearing devices and wearable motion assisting apparatuses, and more particularly to a biological signal measuring wearing device and a wearable motion assisting apparatus for facilitating attachment of a biological signal sensor for obtaining a biological signal from a human body.

### BACKGROUND ART

Motions easy for typical people to make are often very difficult to physically-challenged people or elderly people. Therefore, nowadays, various power-assisted apparatuses (motion assisting apparatuses) for assisting motions of these people or making the motions for them are developed by the inventor of the present invention and so forth. (See, for example, Patent Document 1.)

According to the technique illustrated in Patent Document 1, the motion assisting apparatus is an apparatus configured to assist the walking motions of people challenged in their lower limb motor function, who, for example, have difficulty in walking because of a decrease in the muscular strength of their skeletal muscles, or people having difficulty in walking independently, such as patients undergoing walking motion rehabilitation. This motion assisting apparatus is so operable as to detect a biological signal generated at the time of generating muscular strength in response to a signal from the brain (for example, a surface electro myogram/myoelectricity or the like) and to provide an assisting force from a drive source (for example, an electrically-driven drive motor or the like) based on this detection signal.

### [Prior-Art Document]

### [Patent Document]

[Patent Document 1] Japanese Laid-Open Patent Application No. 2006-204426

### SUMMARY OF THE INVENTION

### PROBLEMS TO BE SOLVED BY THE INVENTION

In such a wearable motion assisting apparatus, control is performed based on bioelectric potential signals of a wearer in order to realize an assist in which the wearer's intention is reflected. In the wearable motion assisting apparatus, multiple biological signal obtaining parts such as electrodes are attached directly to target body parts in order to obtain these bioelectric potential signals, so that it takes time and effort to attach or detach the biological signal obtaining parts every time the wearable motion assisting apparatus is put on or taken off. Further, when it is necessary to attach the biological signal obtaining parts to parts where the wearer cannot attach them by her/himself, the wearer has to get assistance from another person (helper). Further, for the helper as well, it takes time and effort to find the best attachment positions and attach the biological signal obtaining parts every time.

The present invention is made in view of the above-described problems and has an object of providing a biological signal measuring wearing device and a wearable motion assisting apparatus for facilitating attachment of a biological signal sensor for obtaining a biological signal from a human body.

### MEANS FOR SOLVING THE PROBLEMS

In order to solve the above-described problems, the present invention employs means for solving the problems with the following features.

According to the present invention, a biological signal measuring wearing device configured to measure a biological signal from a body surface of a wearer includes a wearing device body configured to cover the body surface of the wearer; a biological signal detecting part provided on a predetermined part of an inner surface of the wearing device body and configured to detect the biological signal from the body surface of the wearer; and a signal communicating part configured to output the signal detected by the biological signal detecting part.

This makes it possible to easily put on the biological signal detecting part for obtaining a biological signal from a human body.

Further, according to the present invention, a biological signal obtaining part is provided that is configured to obtain the biological signal from the signal detected by the biological signal detecting part.

This makes it possible to extract signals used for control (such as an electro myogram/myoelectricity signal and a neurotransmission signal) from the biological signal detected by the biological signal detecting part. This makes it possible to prevent mixture of noise due to signal output, for example.

Further, according to the present invention, multiple biological signal detecting parts are provided, and the biological signal obtaining part is provided for each of the individual biological signal detecting parts or for each of biological signal detecting part groups each of a preset number of the biological signal detecting parts.

This makes it possible to obtain the biological signals in the biological signal detecting parts independent of each other, and to obtain multiple biological signals together.

Further, according to the present invention, multiple biological signal detecting parts are provided, and the signal communicating part is provided for each of the individual biological signal detecting parts or for each of biological signal detecting part groups each of a preset number of the biological signal detecting parts.

This makes it possible to transmit the biological signals, obtained on a biological signal detection part basis, independent of each other, and to transmit multiple biological signals together.

Further, according to the present invention, the wearing device body is formed of a stretchable material to come into contact with the body surface and has an opening part provided in at least a part thereof, the biological signal detecting part is placed so as to allow the biological signal from the body surface to be detected via the opening part, and the signal communicating part is configured to transmit the biological signal detected by the biological signal detecting part by a wired or radio communication.

This makes it possible to easily put on the biological signal detecting part for obtaining a biological signal from a human body.

Further, according to the present invention, a net member woven from a stretchable material is provided between the opening part and the biological signal detecting part.

This prevents the biological signal detecting part from directly coming into close contact with the skin of the wearer. Accordingly, at the time of putting on the biological signal measuring wearing device, the biological signal detecting part is prevented from being sticky, thus making it possible to attach or detach the biological signal detecting part with ease.

Further, according to the present invention, the biological signal obtaining part is configured to obtain the biological signal by causing a portion thereof rising through mesh openings of the woven net member to come into close contact with the body surface of the wearer.

For example, an excessively high adhesive force of the biological signal detecting part may make it difficult for the wearer to put on or take off the wearing device body. Therefore, by thus causing the biological signal detecting part to come into close contact with the body surface of the wearer at multiple fine points using the mesh openings, it is possible to facilitate putting on or taking off the wearing device body while ensuring a good electrical connection.

Further, according to the present invention, the attachment part includes a mark part configured to allow the biological signal detecting part or the net member to be fixed at a predetermined position.

By thus providing the mark part to serve as a mark, it is possible to place the biological signal detecting part and the net member at predetermined positions with ease and accuracy. This makes it possible to exactly measure a biological signal with higher accuracy.

Further, according to the present invention, the wearing device body is an inner suit, a legging, a supporter, a glove, or a sock.

By thus using an existing garment having adhesiveness for the wearing device body, it is possible to measure a biological signal from the skin of the wearer with ease.

Further, according to the present invention, a wearable motion assisting apparatus is configured to control driving of a motion assisting wearing device having a drive source configured to provide a wearer with power, and to assist a motion of the wearer or make the motion for the wearer, based on a biological signal obtained from the above-described biological signal measuring wearing device.

By thus controlling the driving of the motion assisting wearing device having the drive source configured to provide the wearer with power based on a biological signal obtained from the biological signal measuring wearing device, it is possible to smooth and optimize a series of motions desired by the wearer in accordance with a situation at the time.

### EFFECTS OF THE INVENTION

According to the present invention, it is possible to facilitate attachment of a biological signal sensor for obtaining a biological signal from a human body.

### BRIEF DESCRIPTION OF THE DRAWINGS

FIG. 1A is a front view of a biological signal measuring wearing device in which the present invention is implemented.
FIG. 1B is a rear view of the biological signal measuring wearing device in which the present invention is implemented.
FIG. 2A is a diagram illustrating another example (a glove) of the biological signal measuring wearing device.
FIG. 2B is a diagram illustrating another example (a sock) of the biological signal measuring wearing device.
FIG. 2C is a diagram illustrating another example (a supporter) of the biological signal measuring wearing device.
FIG. 3 is a diagram showing an example for illustrating a specific configuration around an electrode part of the biological signal measuring wearing device.
FIG. 4 is an exploded perspective view of individual components around the electrode part illustrated in FIG. 3.
FIG. 5 is a cross-sectional view illustrating attachment of the electrode part and its neighborhood to a wearer.
FIG. 6 is a diagram illustrating a variation of the biological signal measuring wearing device according to a first embodiment.
FIG. 7 is a diagram illustrating a first functional block configuration in the biological signal measuring wearing device.
FIG. 8 is a diagram illustrating a second functional block configuration in the biological signal measuring wearing device.
FIG. 9 is a diagram illustrating a wearable motion assisting apparatus to which the biological signal measuring wearing device is applied.
FIG. 10 is a schematic diagram illustrating the exterior of a biological signal measuring wearing device having biological signal sensor groups on a lower-body (legs) wearing device body.
FIG. 11 is a diagram illustrating a measuring module (a biological signal obtaining part) connected to a biological signal sensor group provided on the wearing device body.
FIG. 12 is a diagram illustrating a biological signal distribution using the biological signal measuring wearing device.
FIG. 13A is an exploded perspective view illustrating an outline of the configuration of a biological signal sensor.
FIG. 13B is a schematic enlarged view of a cross section of the biological signal sensor in an assembled state.

### DESCRIPTION OF EMBODIMENTS

### [About the Present Invention]

The present invention relates to wear for biological signal measuring that makes it possible to reduce the trouble of a wearer or a helper directly attaching or detaching multiple biological signal detecting parts (biological signal sensors) one by one and reducing a load on a wearer by causing the wearer to put on a biological signal measuring wearing device having, for example, biological signal sensors such as electrodes provided at predetermined positions in order to measure biological signals obtainable from the human body of the wearer. Further, by using the biological signal measuring wearing device according to the present invention, it is possible to avoid forgetting to attach a biological signal sensor or connecting wrong interconnects to biological signal sensors.

Further, as a result of using the biological signal measuring wearing device according to the present invention, by, for example, causing a wearer to first put on the biological signal measuring wearing device and then put on a motion assisting wearing device having a drive source to provide the wearer with power over the biological signal measuring wearing device, it is possible to transmit biological signals obtained from the biological signal measuring wearing device, such as the bioelectric potential signals of predetermined parts of the wearer, to the wearable motion assisting apparatus and to control the wearable motion assisting apparatus based on these bioelectric potential signals so as to assist motions of the wearer or make the motions for the wearer by driving the drive source provided on the wearable motion assisting apparatus.

The biological signal measuring wearing device described below includes, as a wearing device body, a wearing device body configured to come into close contact at least partially with the body surface of the wearer and cover the body surface. This wearing device body may be like, for example, a pair of pants or a shirt (a garment for a part on which the wearable motion assisting apparatus is worn) or a supporter that is attached closely to a joint of a human body to protect or support the joint. Further, the wearing device body may also have such a structure as to cause the entire wearing device body to come into close contact with the body surface, such as a so-called "inner suit," or allow only the part of biological signal sensors to come into close contact with the body surface although being structured to be less likely to adhere to the body surface as a whole.

Biological signal detecting parts configured to detect biological signals from the body surface of the wearer are provided on the inner surface of this wearing device body, that is, the surface to contact the skin surface of the wearer with the wearer wearing the wearing device body. In this embodiment, electrodes configured to detect potential signals from the body surface of the wearer are provided as the biological signal detecting parts (biological signal sensors). Electrically conductive adhesive surfaces are formed on the surfaces of the electrodes on the side to contact the body surface of the wearer so as to allow the electrodes to come into close contact with the skin surface of the wearer to measure potentials at the skin surface of the wearer.

Further, according to the present invention, the wearing device body includes biological signal obtaining parts (filters), so that predetermined signals (such as electro myogram/myoelectricity signals and neurotransmission signals) may be extracted from the biological signals detected with the biological signal sensors and be output. This makes it possible to, for example, prevent degradation of signals due to mixture of noise at the time of signal transmission.

A description is given in detail below, using drawings, of embodiments where a biological signal measuring wearing device and a wearable motion assisting apparatus in the present invention with the features as described above are suitably implemented.

In the present invention, biological signals are signals originating from the biological activities of a wearer, and are signals that are measurable from a body and also vary in time series. Specifically, biological signals include, for example, potentials generated by biochemical reactions such as electro myogram/myoelectricity signals, neurotransmission signals, brain waves, cardiac potentials, and potentials generated by motion artifacts (the effects of motions); and signals generated by biological activities such as vibrations such as pulse waves generated by heart beatings.

In the embodiments illustrated below, an electrode is used, by way of example, as a biological signal detecting part (a biological signal sensor) configured to detect a biological signal from the body surface of a wearer, and a bioelectric potential signal, which is an electrical signal detected through the electrode attached to the body surface of the wearer, is used as the biological signal.

### [First Embodiment]

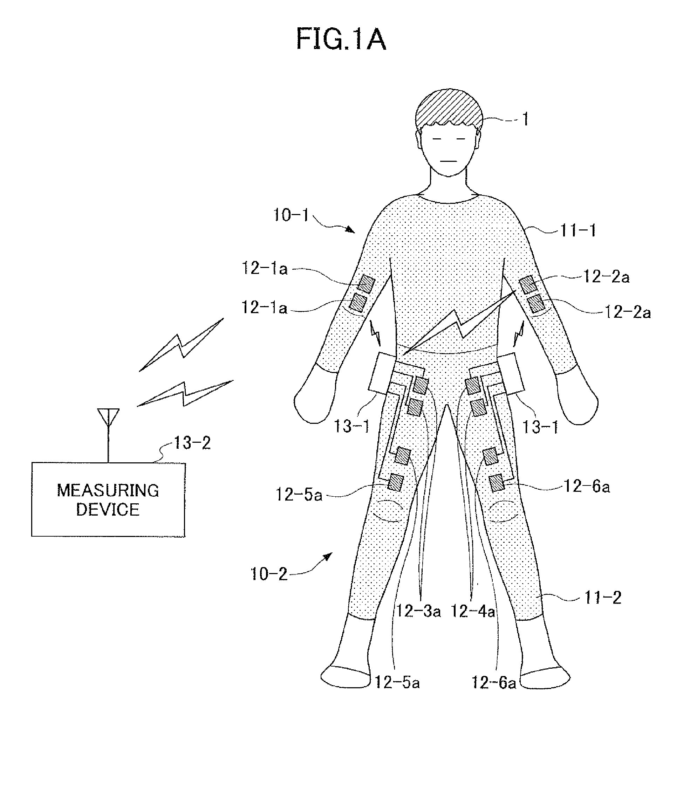

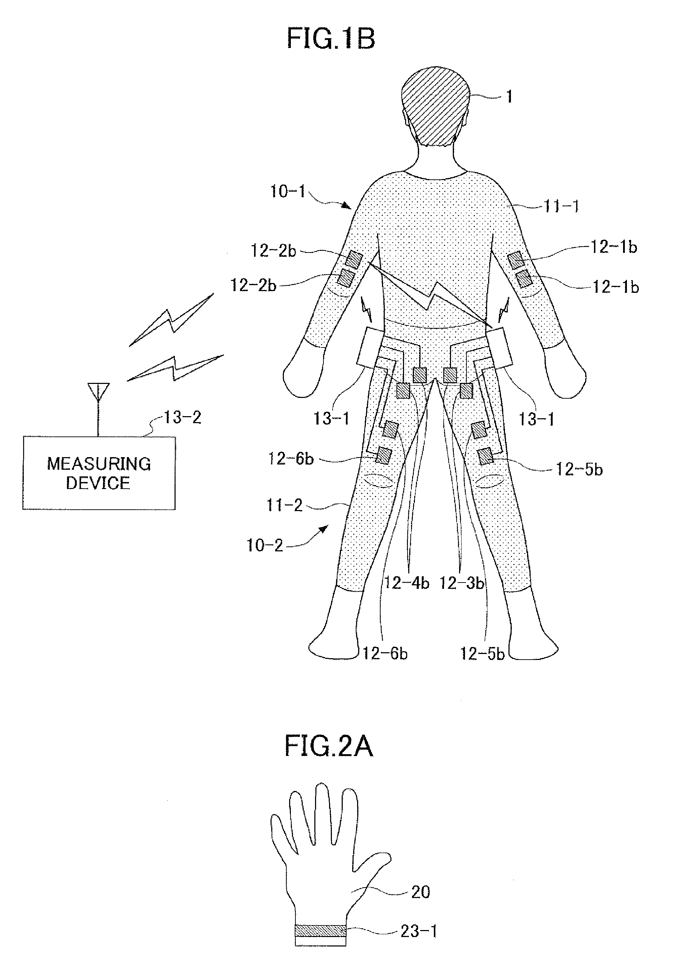

First, a description is given of a biological signal measuring wearing device where the present invention is implemented. FIG. 1A is a front view of the biological signal measuring wearing device in which the present invention is implemented. Further, FIG. 1B is a rear view of the biological signal measuring wearing device in which the present invention is implemented. The drawings shown in FIGS. 1A and 1B illustrate a case where a wearer 1 wears an upper-body biological signal measuring wearing device 10-1 and a lower-body biological signal measuring wearing device 10-2.

A biological signal measuring wearing device according to this embodiment includes a wearing device body configured to cover the body surface of a wearer and be put on the wearer, and has a biological signal sensor (an electrode part) configured to detect a bioelectric potential signal as a biological signal sensor at at least one position where a biological signal from the wearer is measureable on the inner surface of this wearing device body (a surface to contact the body surface of the wearer with the wearing device body attached to the wearer). Further, the biological signal measuring wearing device includes a signal transmitting part configured to transmit a biological signal (such as an electro myogram/myoelectricity) obtained from the body of the wearer using the electrode part to an external apparatus such as a measuring unit by wired or radio transmission.

Being more specific, as illustrated in FIGS. 1A and 1B, the upper-body biological signal measuring wearing device 10-1 worn by the wearer 1 has, on the inner surface of a wearing device body 11-1, a pair of right-side electrode parts 12-1a and a pair of left-side electrode parts 12-2a as bioelectric potential sensors on the elbow-joint flexor side provided around the biceps brachii of both arms and a pair of right-side electrode parts 12-1b and a pair of left-side electrode parts 12-2b as bioelectric potential sensors on the elbow-joint extensor side provided around the triceps brachii of both arms. Thus, a bioelectric potential sensor is composed of a pair of electrode parts, and is configured to detect a bioelectric potential signal of a wearer by detecting a difference in potential between the electrode parts.

Further, as illustrated in FIGS. 1A and 1B, the lower-body biological signal measuring wearing device 10-2 worn by the wearer 1 has, on the inner surface of a wearing device body 11-2, a pair of right-side electrode parts 12-3a and a pair of left-side electrode parts 12-4a as bioelectric potential sensors on the hip-joint flexor side provided around the long adductor muscles or the iliopsoas muscles of both legs; a pair of right-side electrode parts 12-3b and a pair of left-side electrode parts 12-4b as bioelectric potential sensors on the hip-joint extensor side provided around the gluteus maximus muscle of both legs; a pair of right-side electrode parts 12-5a and a pair of left-side electrode parts 12-6a as bioelectric potential sensors on the knee-joint extensor side provided around the quadriceps femoris muscles of both legs; and a pair of right-side electrode parts 12-5b and a pair of left-side electrode parts 12-6b as bioelectric potential sensors on the knee-joint flexor side provided around the biceps femoris muscles of both legs.

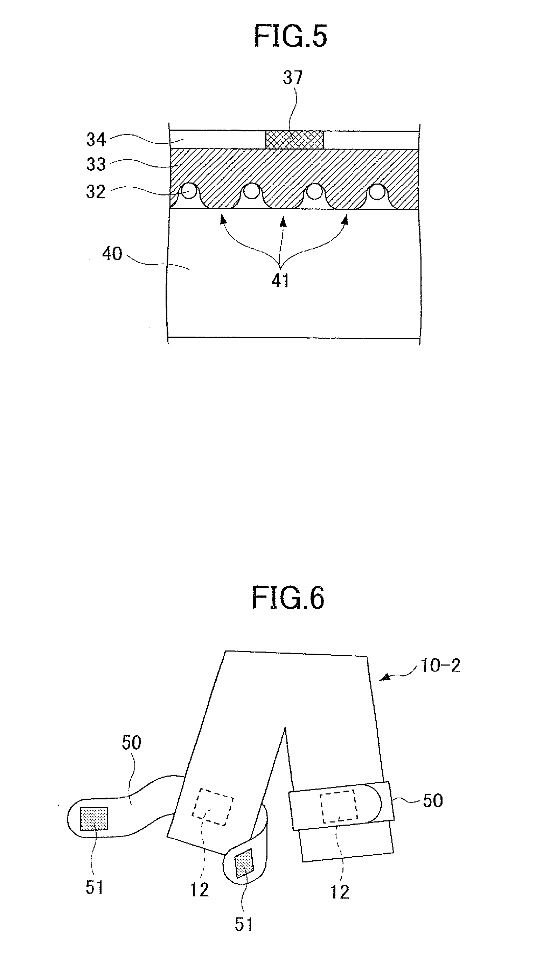

Here, the wearing device bodies 11-1 and 11-2 of the biological signal measuring wearing devices 10-1 and 10-2 illustrated in FIGS. 1A and 1B are formed of a stretchable material (whose examples include elastic materials and those so woven [of such fabrics] as to be stretchable) so as to come into close contact with the body surface of the wearer 1. Examples of the wearing device bodies 11-1 and 11-2 include so-called "inner wear," supporters, leggings, stockings, taping tights, and garments for supporting or assisting motions of a wearer in exercises. Further, aside from the form illustrated in FIGS. 1A and 1B, the wearing device bodies 11-1 and 11-2 may also be garments that fit less tightly to a body surface or garments so formed as to not tighten a body, such as so-called "underwear." In the case of applying such less tight-fitting garments to the wearing device bodies 11-1 and 11-2, providing a configuration to moderately press only parts where biological signal sensors are provided with bands or the like (see, for example, FIG. 6 described below) is preferable because this allows the biological signal sensors to adhere to the body surface of a wearer. Further, the wearing device body may be anything that is wearable by a wearer in such a manner as to cover her/his body surface. Specifically, as illustrated in FIGS. 1A and 1B, the biological signals obtained with the respective bioelectric potential sensors described above are output from the respective electrode parts 12-1a through 12-6a and 12-1b through 12-6b to measuring devices 13-1 and 13-2. The signals output from the electrode parts 12-1a through 12-6a and 12-1b through 12-6b, which are biological signal sensors, to the measuring devices 13-1 and 13-2 have address information (installation position information) for identifying the respective signals.

In this case, the biological signals are transmitted from the electrode parts 12-1a through 12-6a and 12-1b through 12-6b to the measuring devices 13-1 and 13-2 by wired communications or radio communications. In FIGS. 1A and 1B, the lower-body wearing device body 11-2 illustrates a case of wired communications and the upper-body wearing device body 11-1 illustrates a case of radio communications. In the case of wired communications, the electrode parts 12-3a through 12-6a and 12-3b through 12-6b and the measuring devices 13-1 are connected to each other with interconnects formed of electrically conductive wires such as leads. These interconnects may be provided inside or outside the biological signal measuring wearing device 10-2. Alternatively, interconnects like strand wires may be provided by being sewn onto the biological signal measuring wearing device 10-2.

In the case of radio, radio communications are performed with a transmitting circuit, a receiving circuit, an antenna and the like provided in each of the electrode parts 12-1a, 12-2a, 12-1b, and 12-2b and the measuring devices 13-1, so that signals including biological signals are transmitted and received. A radio tag in this embodiment, which includes an integrated circuit (IC) chip with an antenna processed into a tag or label shape, for example, is a noncontact information transmitting method that transmits a biological signal detected in the electrode part and address information stored in its IC via the antenna and receives signals from the measuring devices 13-1 and 13-2 via the antenna.

The measuring devices 13-1 and 13-2 have the function of transmitting a data obtaining start signal, a data obtaining end signal, etc., the function of storing data, an antenna for receiving data transmitted by radio, and a connector configured to connect to a wire to receive information from the wire.

This allows the measuring devices 13-1 illustrated in FIGS. 1A and 1B to obtain data by wired or radio communications from the electrode parts 12-1a through 12-6a and 12-1b through 12-6b. For example, the measuring device 13-1 receives biological signals transmitted from the radio tags of the electrode parts 12-1a, 12-2a, 12-1b, and 12-2b provided on the upper-body biological signal measuring wearing device 10-1. Further, the measuring device 13-1 receives biological signals from the electrode parts 12-3a through 12-6a and 12-3b through 12-6b provided on the lower-body biological signal measuring wearing device 10-2 via electrically conductive wires.

Further, the measuring device 13-2 illustrated in FIGS. 1A and 1B receives biological signals transmitted by the radio tags from the electrode parts 12-1a through 12-6a and 12-1b through 12-6b provided on the biological signal measuring wearing devices 10-1 and 10-2.

Here, the measuring device 13-1 is a unit that mainly correlates biological signals obtained through, for example, filters (a biological signal obtaining part) or the like from signals detected by the electrode parts 12-1a through 12-6a and 12-1b through 12-6b, which are biological signal sensors, with the addresses (installation position information) of the sensors and stores the biological signals correlated with the addresses in a memory (a storage part) provided in advance in the measuring device 13-1, or transmits the biological signals to an external terminal or the like such as the measuring device 13-2 through a communications part (a signal communicating part).

On the other hand, the measuring device 13-2 is an external unit that mainly determines an apparatus (an external apparatus) connected via an input/output part provided in advance in the measuring device 13-2, and further converts biological signals input via a communications part (a signal communicating part) from the measuring device 13-1 or the electrode parts 12-1a through 12-6a and 12-1b through 12-6b into a format corresponding to the kind (machine type and hardware configuration [a display unit such as a display, an output unit such as a printer, a wearable motion assisting apparatus, etc.]) of the apparatus and outputs the converted biological signals.

The measuring device 13-1 may have the function of the measuring device 13-2, or the measuring device 13-2 may have the function of the measuring device 13-1.

That is, the measuring devices 13-1 and 13-2 may store the obtained biological signals in correlation with the installation position information of the electrode parts 12-1a through 12-6a and 12-1b through 12-6b provided on the biological signal measuring wearing devices 10-1 and 10-2. Further, the measuring devices 13-1 and 13-2 may manage all of the electrode parts 12-1a through 12-6a and 12-1b through 12-6b by storing the obtained signals in correlation with the position information of the electrode parts 12-1a through 12-6a and 12-1b through 12-6b. Further, it is possible to have the motion of a predetermined part of the wearer mechanically assisted or have the motion mechanically made for the wearer by, for example, driving an actuator of a wearable motion assisting apparatus described below based on the biological signals from the electrode parts 12-1a through 12-6a and 12-1b through 12-6b stored in the measuring device 13-2 or the like.

The positions, the size, or the number of the electrode parts 12-1a through 12-6a and 12-1b through 12-6b are not limited to those of the case illustrated in FIGS. 1A and 1B. Further, the biological signal measuring wearing devices 10-1 and 10-2 illustrated in FIGS. 1A and 1B are upper and lower separate bodies. In the present invention, however, the biological signal measuring wearing devices 10-1 and 10-2 are not limited to this, and may be formed as a unit, for example.

### [Other Examples of Biological Signal Measuring Wearing Device according to First Embodiment]

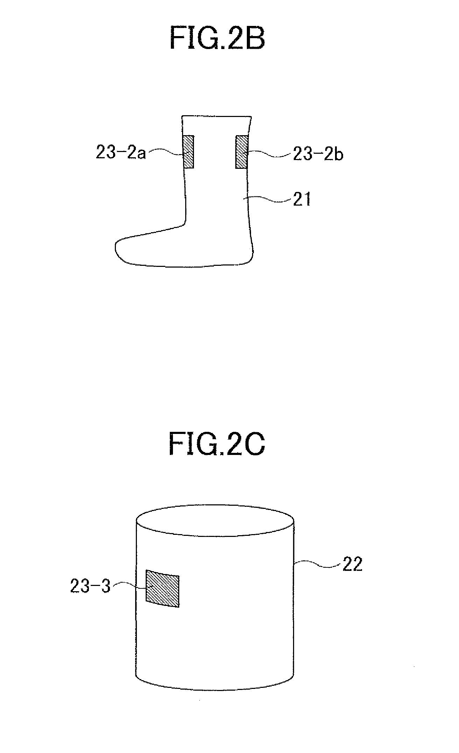

Next, a description is given of examples where the biological signal measuring wearing device according to the first embodiment is applied to parts other than the legs. FIG. 2A is a diagram illustrating another example (a glove) of the biological signal measuring wearing device. Further, FIG. 2B is a diagram illustrating another example (a sock) of the biological signal measuring wearing device. FIG. 2C is a diagram illustrating another example (a supporter) of the biological signal measuring wearing device.

Here, a glove 20 illustrated in FIG. 2A has a pair of electrode parts 23-1 provided around, for example, a wrist. Further, a sock 21 illustrated in FIG. 2B has a pair of electrode parts 23-2a and a pair of electrode parts 23-2b provided around, for example, the extensor digitorum longus muscle, the anterior tibial muscle, and the triceps surae muscle. Further, a supporter 22 illustrated in FIG. 2C is a supporter (assisting device) to be wrapped around a predetermined part of the wearer 1. An electrode part 23-3 is formed at such a position as to allow a biological signal to be obtained from the predetermined part by wrapping the supporter 22 around it. It is preferable that the biological signal measuring wearing devices 10 be worn in (close) contact with the body surface (skin) of the wearer 1.

With respect to the electrode parts 23 illustrated in FIGS. 2A through 2C, at least one pair may be provided at such a position as to allow a biological signal to be obtained as required. Accordingly, specifically, in the case of a hand of the wearer 1, since there are normally the long palmar muscle, the tendon of the flexor carpi radialis muscle, the tendon of the flexor carpi ulnaris muscle, the tendons of the flexor digitorum superficialis muscle, and the tendons of the flexor digitorum profundus muscle on the palm side of a wrist, and the flexion of the wrist is caused by the contraction of the long palmar muscle and the tendon of the flexor carpi radialis muscle and the flexion of fingers is caused by the contraction of the tendons of the flexor digitorum superficialis muscle and the tendons of the flexor digitorum profundus muscle, the biological signal measuring wearing device according to the present invention may have an electrode part provided at such a position as to allow at least one biological signal to be obtained among these.

### [About Electrode Part]

Here, the above-described electrode parts 12 and 23 in this embodiment are electrode parts configured to obtain biological signals, and are preferably adhesive or elastic like rubber or gel or in the form of a sheet in order to measure biological signals appearing on the skin surface of the wearer 1 in particular. This makes it possible to increase adhesion between the wearer 1 and the body surface at the electrode parts 12 provided on the biological signal measuring wearing devices 10.

For example, in the case of rubber-like elastic electrode parts, it is possible to increase adhesion between the electrode parts 12 and 23 and the skin surface of the wearer 1. Further, if the electrode parts 12 and the like have sponge-like elasticity in the directions of thickness relative to the surfaces of the electrode parts 12, pressing the electrode parts 12 against the wearer 1 causes the electrode parts 12 to deform in accordance with the irregularities of the body shape of the wearer 1, so that it is possible to increase adhesion in accordance with the body shape of the wearer 1.

Further, sheet-shaped electrically conductive gel, electrically conductive fibers, etc., may be applied to the electrode parts 12 and 23, which may be, for example, polyamide synthetic fibers or polyester or cotton fibers having an electrically conductive material such as metal adhered to their surfaces. Further, examples of the metal used may include copper and aluminum.

The electrode parts 12 and 23 may be configured otherwise as long as the electrode parts 12 and 23 are electrically conductive, low in contact resistance due to contact with the body surface of the wearer 1, and harmless to human bodies. Further, the electrode parts 12 and 23 may partially include fibers that are not electrically conductive.

### [Specific Configuration around Electrode Part of Biological Signal Measuring Wearing Device]

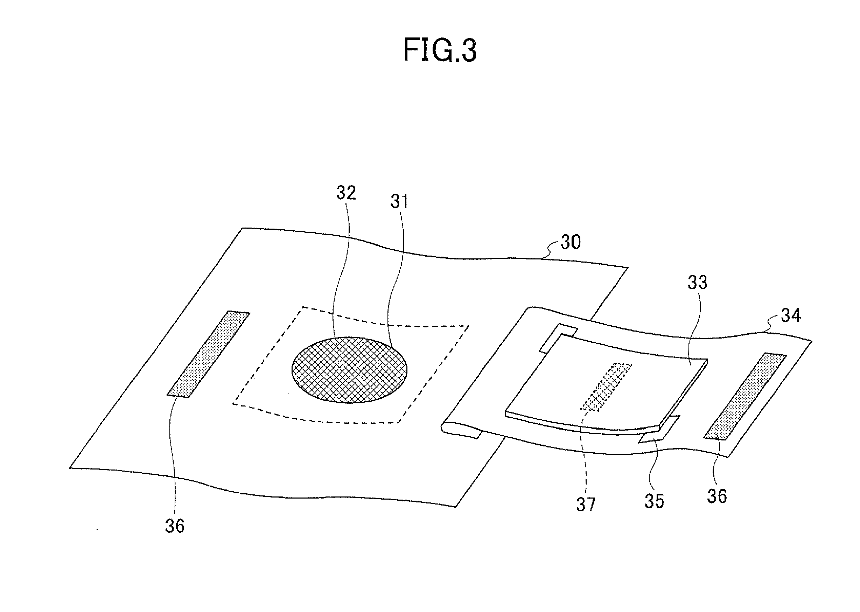

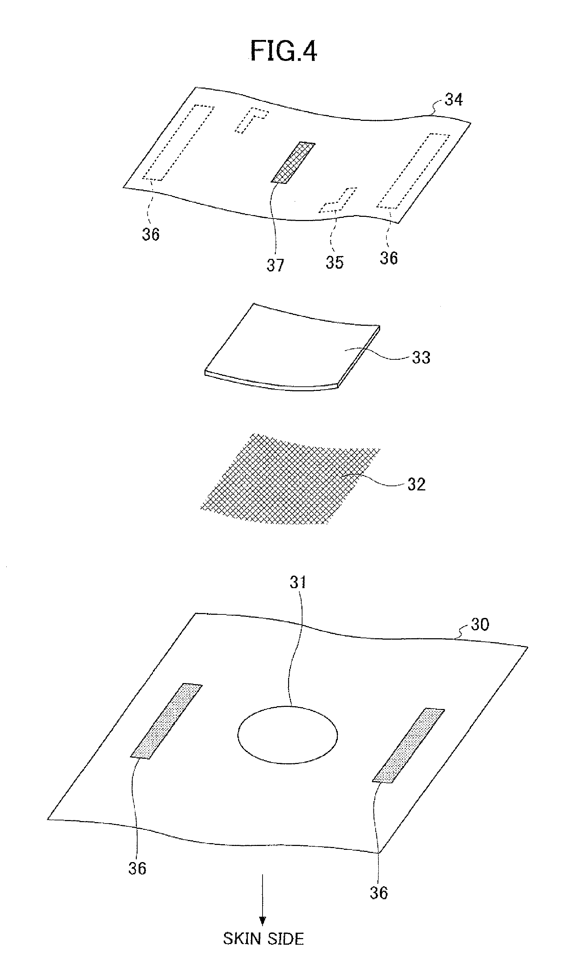

Next, a description is given, using drawings, of a specific configuration around an electrode part of the biological signal measuring wearing devices 10. FIG. 3 is a diagram showing an example for illustrating a specific configuration around an electrode part of the biological signal measuring wearing device. Further, FIG. 4 is an exploded perspective view of individual components around the electrode part illustrated in FIG. 3.

For example, as illustrated in FIG. 3 and FIG. 4, the biological signal measuring wearing device is configured to include, around an electrode part, a wearing device body 30 such as a pair of leggings, an opening part 31, a net member 32, an electrode pad 33, a cover member 34, mark parts 35, attachment parts 36, and a radio tag part 37 as a signal communicating part.

As illustrated in FIG. 3 and FIG. 4, the wearing device body 30 is formed of a stretchable material (whose examples include elastic materials and those so woven [of such fabrics] as to be stretchable). Further, the wearing device body 30 is provided with the opening part 31, through which the electrode pad 33 may be in direct contact with the skin of the wearer 1. Therefore, it is possible to obtain a biological signal from the wearer 1 with accuracy in a low noise condition. Further, the net member 32 is provided over the opening part 31 as a window part. In the present invention, the opening part 31 is not limited to a particular shape, size, etc., and, for example, may have any of a circular shape, an elliptical shape, a rectangular shape, a triangular shape, a rhombic shape, etc., depending on which part of the skin of the wearer 1 to contact.

By thus providing the opening part 31 with the net member 32, a set of fine holes due to the mesh of the net member 32 is formed in the opening part 31. Accordingly, when the electrode pad 33 is placed on the opening part 31 (for example, a set of fine holes or the like), the electrode pad 33 elastically deforms to rise on the rear side (the side contacting the body surface of the wearer 1) of the wearing device body 30 through the fine holes. As a result, the electrode pad 33 and the body surface of the wearer 1 are in contact at multiple fine points. Here, in order to obtain a biological signal with accuracy, it is necessary to ensure that the electrode pad 33 is in close contact with the body surface of the wearer 1 to ensure a good electrical connection. However, an excessively high adhesive force of the electrode pad 33 may make it difficult for the wearer 1 to put on or take off the wearing device body 30. Therefore, by thus causing the electrode pad 33 to come into contact with the body surface of the wearer 1 at multiple fine points, it is possible to facilitate putting on or taking off the wearing device body 30 while ensuring a good electrical connection.

It is preferable that the net member 32 be stretchably woven from, for example, an insulating resin material, an electrically conductive metal, etc., so as to be formed with elasticity. The shape and size of mesh openings are not limited in particular, and may be adjusted so that the rising portions of the electrode pad 33 coming out of the mesh openings of the net member 32, generated with the net member 32 in response to an external pressure applied to the electrode pad 33, come into close contact with the body surface (skin) of the wearer 1. Alternatively, multiple holes may be formed in a thin sheet of such size as to cover the opening part 31. That is, the net member 32 may be so configured as to have multiple small holes formed with the opening part 31.

Further, the net member 32 may be attached to and formed unitarily with the wearing device body 30, or may be attached to the electrode pad 33. Here, a general-purpose electrode pad may be applied as the electrode pad 33 in the case where the net member 32 is attached to the wearing device body 30. Further, it is possible to wash the wearing device body 30 with ease in the case where the net member 32 is attached to the electrode pad 33.

Further, for example, an adhesive, elastic gel pad may be used as the electrode pad 33. The gel hardness of the electrode 33 may be changed as desired by selecting an intermediate base depending on the position of adhesion to the wearer 1 or the preference of the wearer 1. Examples of the intermediate base may include a polyester intermediate base of a large fiber diameter. This allows the base itself to have tension so that the electrode pad 33 may be hard finished as a gel product. Further, in the case of using, for example, light, thin non-woven polyester cloth as the intermediate base, the electrode pad 33 becomes soft as a gel product because of high flexibility of the base, so that it is possible to cause the electrode pad 33 to adhere to the uneven body surface of the wearer 1 with accuracy. For example, a general-purpose medical electrode pad or the like may be used as the electrode pad 33.

Not only one but also two or more electrode pads 33 may be arranged in a predetermined part. Further, the shape of the electrode pad 33 is formed in conformity to the size and shape of the opening part 31. Further, the placement of the electrode pad 33 may be so determined as to allow the electrode pad 33 to come into close contact with the wearer 1 through the opening part 31 to detect a biological signal. It is preferable that the electrode pad 33 be thin and light.

Further, the cover member 34 is configured to cover the wearing device body 30, the net member 32, and the electrode pad 33 all together. It is preferable that the cover member 34 sufficiently press other components, which makes it possible to increase the degree of adhesion to the wearer 1. Accordingly, the cover member 34 is formed of, for example, a cloth material or an elastic rubber material. The presence of this cover member 34 makes it possible to facilitate fixation of the electrode pad 33 and to protect the electrode pad 33 with the cover member 34 without displacement of the electrode pad 33.

Here, in assembling the above-described individual components around the electrode part illustrated in FIG. 3 and FIG. 4 into a unit, first, the electrode pad 33 is positioned based on marks such as the mark parts 35, at least one of which is provided as a mark, on the bottom surface of the cover member 34. The mark parts 35 may serve to position the net member 32 in the same manner. The positions, kind, number, etc., of mark parts 35 are not limited in particular in the present invention. The mark parts 35 may be any of an L letter, a crisscross, a point, a circle, a star, a rectangle, a polygon, etc., in shape in accordance with a condition such as aligning with end parts or corner parts of the electrode pad 33 corresponding to the preset size of the electrode pad 33 or aligning with the center of the electrode pad 33.

Further, in order to allow fixation of the electrode pad 33 or the net part 32, the mark parts 35 may be provided with, for example, irregularities or opening parts to allow end parts of the electrode pad 33 to be inserted and fixed.

Further, in the above-described embodiment, the mark parts are provided on the cover member 34. The present invention, however, is not limited to this, and the mark parts 35 may alternatively be provided on, for example, the wearing device body 30.

Here, fixing the cover member 34 to the wearing device body 30 includes attaching the opening part 31, the net member 32, the electrode pad 33, and the cover member 34 relative to each other in a sandwiching manner using the attachment part 36. At this point, it is preferable that the attachment part 36 attaches the net member 32 and the electrode pad 33 in such a manner as to increase adhesion to the skin of the wearer 1.

Here, the attachment part 36 may employ one or a combination of two or more of, for example, Magic Tape (registered trademark) (a hook-and-loop fastener), a zipper (fastener), a snap fastener, an eyehook, a button, magnets, double-sided tape, etc. Further, for example, FIG. 4 illustrates the case where the attachment part 36 is Magic Tape (registered trademark). The attachment part 36 of a predetermined region is provided on the wearing device body 30 and the cover member 34. Hooks catch in loops because of their respective shapes so as to fasten together to allow attachment.

Further, for example, as illustrated in FIG. 3, the wearing device body 30 and the cover member 34 are fixed by sewing them together on one side (sewing one end of the cover member 34), etc. However, the present invention is not limited to this. For example, the wearing device body 30 and the cover member 34 may be configured to be completely attachable and detachable relative to each other.

The above-described configuration allows easy replacement of the net member 32 or the electrode pad 33 and allows washing, etc., to be performed on the wearing device body 30, thus making it possible to further improve usability.

As illustrated in FIG. 3 and FIG. 4, information obtained at the electrode pad 33 may be transmitted by radio to the above-described external apparatus (measuring device, etc.) by the radio tag part 37 to be managed by the external apparatus.

Here, the radio tag part 37 includes a storage part configured to store a predetermined amount of data, such as a memory, a transmitting and receiving part, and an antenna part. The storage part is configured to temporarily store a biological signal obtained from the electrode part 12, and a transmitting and receiving part may perform transmission and reception with the external apparatus via the antenna part and performing wired transmission and reception with the external apparatus. In the present invention, the transmission and reception technique in the transmitting and receiving part is not limited in particular, and may use, for example, electromagnetic coupling, electromagnetic induction, microwaves, and optical communications. Further, the power supply system may be a passive type that receives a supply of electric power from an external apparatus to operate with a battery-less IC or an active type provided with a power supply part in advance.

### [Cross-Sectional View of Biological Signal Measuring Wearing Device 10]

Next, a description is given, using a drawing, of attachment of the electrode part and its neighborhood to the wearer 1. FIG. 5 is a cross-sectional view illustrating attachment of the electrode part and its neighborhood to the wearer 1.

As illustrated in FIG. 5, in the opening part 31 of the wearing device body 30, the electrode pad 33 is allowed to come into contact with a skin 40 (body surface) of the wearer 1 via rising portions 41 of the electrode pad 33 rising through the openings of the net member 32. Accordingly, the electrode pad 33 is prevented from adhering directly to the skin 40. This prevents the electrode pad 33 from being sticky at the time of putting on the biological signal measuring wearing device 10 and facilitates putting or taking off the biological signal measuring wearing device 10. Further, when the adhesive force of one of the electrode pads 33 becomes insufficient, it is possible to replace the one only.

### [Variation of Biological Signal Measuring Wearing Device according to First Embodiment]

Next, a description is given, using a drawing, of a variation of the biological signal measuring wearing device according to the first embodiment. FIG. 6 is a diagram illustrating a variation of the biological signal measuring wearing device according to the first embodiment. In the case illustrated in FIG. 6, the lower-body biological signal measuring wearing device 10-2 includes supporters (assisting devices) 50 as a close-contact supporting member so that electrode parts come into closer contact with the skin of the wearer 1.

As illustrated in FIG. 6, the supporters 50 are wrapped around the wearer 1 at predetermined positions so that the electrode parts 12 come into close contact with the skin of the wearer 1 using attachment parts 51 each including one or a combination of two or more of, for example, Magic Tape (registered trademark) (a hook-and-loop fastener), a zipper (fastener), a snap fastener, an eyehook, a button, magnets, double-sided tape, etc. Specifically, the supporters 50 are wrapped over the corresponding electrode parts 12. Specifically, the attachment parts 51 may be configured to allow the electrode parts 12 to be externally pressed. For example, the attachment parts 51 may be configured to cover corresponding parts using annular elastic members or stretchable fibers such as stockings.

This makes it possible to detect a biological signal with high accuracy by adding a little pressure for better contact when the electrical connection is insufficient. If it is possible to sufficiently press the electrode parts 12 and the skin of the wearer 1 only with the above-described cover member 34, the supporters 50 are unnecessary.

Thus, the above-described biological signal measuring wearing device makes it possible to easily attach electrode parts for measuring biological signals obtainable from a human body. Further, using biological signals obtained from the biological signal measuring wearing device according to the present invention, the results may be displayed with a display part such as a display to applied to an analysis of the biological signals or an analysis of the mechanism of a human body, or be applied to the driving of an actuator of a wearable motion assisting apparatus.

### [Functional Block Configuration in Biological Signal Measuring Wearing Device according to First Embodiment]

Next, a description is given, using drawings, of functional block configurations in the biological signal measuring wearing device according to the first embodiment. For example, the above-described biological signal measuring wearing devices illustrated in FIGS. 1A and 1B or the like may be used for the biological signal measuring wearing device used in the block configurations illustrated below.

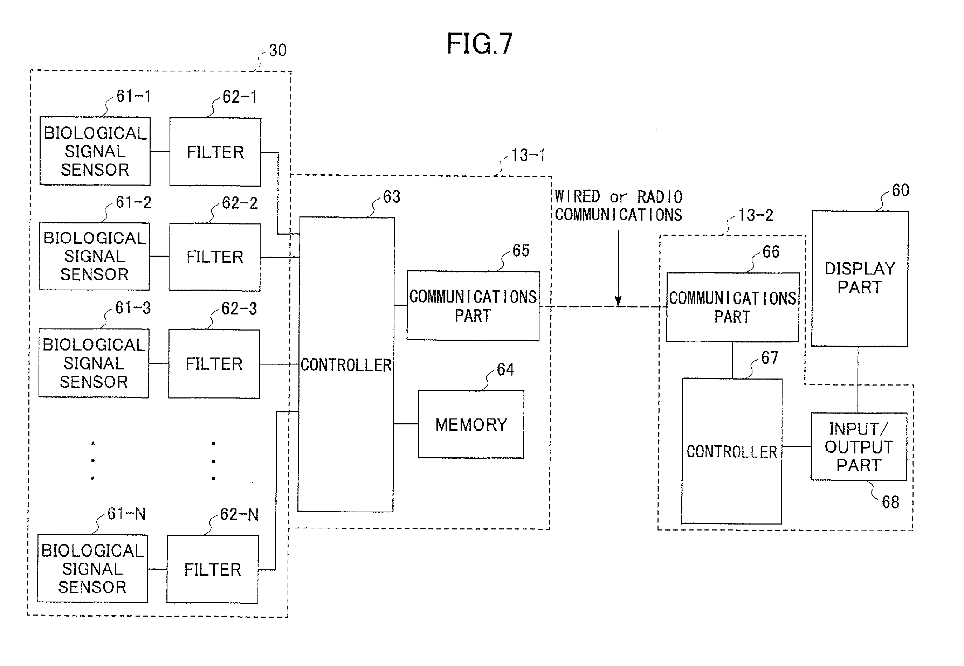

FIG. 7 is a diagram illustrating a first functional block configuration in the biological signal measuring wearing device. The biological signal measuring wearing device illustrated in FIG. 7 includes the wearing device body 30, the measuring device 13-1, and the measuring device 13-2. Further, in FIG. 7, a display part 60 such as a display, which is an example of the external apparatus connected to the measuring device 13-2, is provided.

Further, the wearing device body 30 includes biological signal sensors 61-1 through 61-N (N: an integer greater than or equal to two) such as the above-described electrodes and filters 62-1 through 62-N as biological signal obtaining parts. The above-described biological sensors and filters may be one each in number at the least.

Further, the measuring device 13-1 includes a controller 63 as a control part, a memory 64, and a communications part 65. Further, the measuring device 13-2 includes a communications part 66, a controller 67, and an input/output part 68.

According to the first functional block configuration in the biological signal measuring wearing device illustrated in FIG. 7, the biological signal sensors 61-1 through 61-N are provided with their respective filters 62-1 through 62-N.

The filters 62-1 through 62-N detect biological signals from the corresponding biological signal sensors 61-1 through 61-N in response to a command signal from the controller 63 of the measuring device 13-1 or at a predetermined time. The filters 62-1 through 62-N extract only signals actually needed, such as, for example, electro myogram/myoelectricity signals and neurotransmission signals, from the detected biological signals, and output the extracted signals to the controller 63. Thereby, it is possible to prevent mixture of noise due to signal output, for example.

The controller 63 of the measuring device 13-1 correlates the biological signals obtained through the filters 62-1 through 62-N from the signals detected with the biological signal sensors 61-1 through 61-N with the addresses (installation position information) of the sensors, the date and time of obtaining, etc., and stores the biological signals in the memory 64. The controller 63 may output a command signal for obtaining a biological signal to at least one of the filters 62-1 through 62-N to obtain a biological signal obtained from the biological signal sensor corresponding to the filter.

Further, the controller 63 outputs information on the biological signals including the address information and the date and time information stored in the memory 64 to the measuring device 13-2, which is an external unit, through wired or radio communications via the communications part 65 as a signal communicating part.

The communications part 66 of the measuring device 13-2 receives the biological signals transmitted from the measuring device 13-1, and outputs the received signals to the controller 67. The controller 67 is an external unit that determines an apparatus (the display part 60 or the like in FIG. 7) connected via the input/output part 68 and further outputs the biological signals, etc., obtained via the communications part 66 after converting the biological signals, etc., into such a format as to allow the biological signals, etc., to be output by the above-described display part 60.

Accordingly, the controller 67 may output the biological signals, etc., converted into a predetermined output format to the display part 60 via the input/output part 68. The output destination is not limited to the display part 60. Examples of the output destination may include personal computers, wearable motion assisting apparatuses, and portable recording media such as CD-ROMs, USB (universal serial bus) memories, media cards such as memory sticks, etc.

In the case illustrated in FIG. 7, there is a one-to-one correspondence between the biological signal sensors and the filters. However, the present invention is not limited to this, and for example, a sensor unit or the like may be provided to perform management so that biological signals are obtained from multiple biological signal sensors and output all together to the measuring device 13-1.

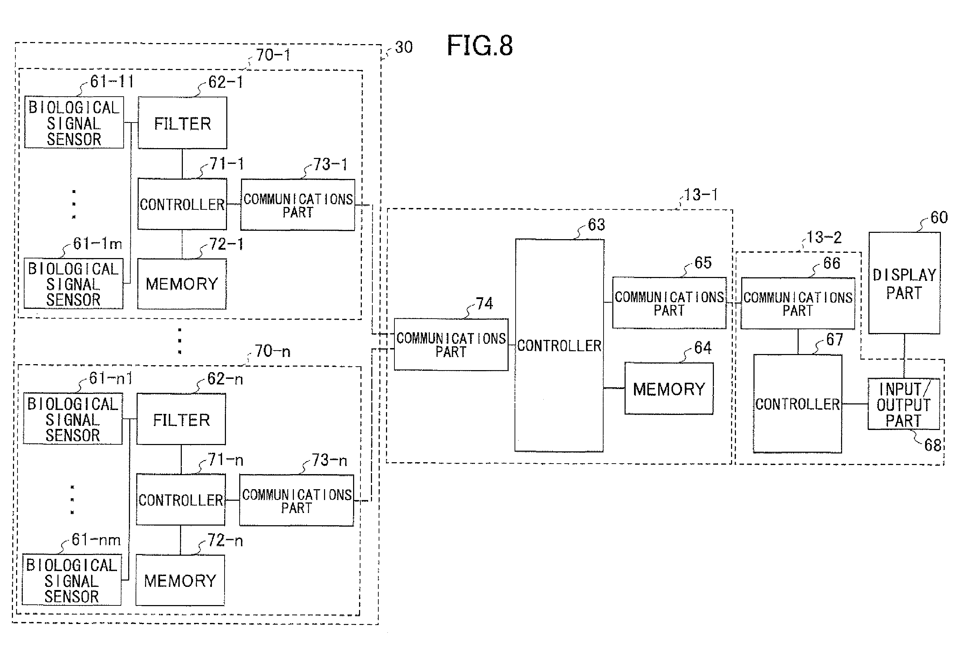

Here, FIG. 8 is a diagram illustrating a second functional block configuration in the biological signal measuring wearing device. In the description of this different configuration of the present invention, the same components as those of the first functional block configuration of the present invention are given the same characters, and a redundant description is omitted.

In the second functional block configuration of the biological signal measuring wearing device illustrated in FIG. 8, the wearing device body 30 is provided with sensor units 70-1 through 70-n (n: an integer greater than or equal to two). Further, the sensor units 70-1 through 70-n includes respective biological signal sensor groups (biological signal detecting part groups) having the same number or different numbers of multiple biological signal sensors (biological signal detecting parts) 61-11 through 61-nm (m: an integer greater than or equal to two); the filters 62-1 through 62-n as biological signal obtaining parts for the respective biological signal sensor groups; respective controllers 71-1 through 71-n as sensor unit controlling parts; respective memories 72-1 through 72-n as storage parts; and respective communications parts 73-1 through 73-n as signal communicating parts. In each of the sensor units 70-1 through 70-n, the number of biological signal sensors may be one at the least.

Further, according to the second functional block configuration illustrated in FIG. 8, a single sensor unit includes a biological signal sensor group formed of multiple biological signal sensors. Further, processing is performed with multiple signal sensors in a single sensor unit being grouped together with a single filter. Further, the filters 62-1 through 62-n are managed by the corresponding controllers 71-1 through 71-n, and obtain biological signals from the biological signal sensors in response to command signals from the corresponding controllers 71-1 through 71-n or at a predetermined time.

The controller 71-1 through 71-n store the biological signals from the filters, along with their respective address information and date and time information, in the corresponding memories 72-1 through 72-n in the sensor units 70-1 through 70-n. Further, the controllers 71-1 through 71-n transmit the biological signals, etc., to a communications part 74 of the measuring device 13-1 by wired or radio communications via the corresponding communications parts 73-1 through 73-n. That is, providing the communications parts 73-1 through 73-n on a biological signal sensor basis or on the basis of a preset number of biological signal sensors allows the biological signals obtained from the biological signal sensors to be transmitted independent of each other as well as in groups of multiple biological signals.

Further, the communications part 74 in the measuring device 13-1 receives the biological signals, etc., transmitted from the communications parts 73-1 through 73-n of the sensor units 70-1 through 70-n, and outputs the received biological signals, etc., to the controller 63. Further, the communications part 74 transmits a command signal received from the controller 63 for obtaining a biological signal to only one or more of the sensor units 70-1 through 70-n for which the communications part 74 has received the command.

This allows multiple biological signal sensors to be managed together by the controller 63 of the measuring device 13-1, so that it is possible to obtain information at locations whose information is needed when the information is needed.

Further, according to this embodiment described above, cases are illustrated where the wearing device body 30 is provided with the filters 62. However, the present invention is not limited to this, and the measuring device 13-1 may be provided with the filters 62.

### [Second Embodiment: Application of Biological Signal Measuring Wearing Device to Wearable Motion Assisting Apparatus]

Next, as a second embodiment, a description is given, using drawings, of a case where the biological signals obtained from the above-described biological signal measuring wearing device are applied to a wearable motion assisting apparatus. In the following description, by way of example, a description is given of a hardware configuration and a drive control technique for providing more natural motions such as walking, standing, and sitting with HAL (Hybrid Assistive Limb), a wearable exoskeletal power-assisted system, in order to assist or reconstruct the lower-body motor function through biped walking.

A wearable motion assisting apparatus in this embodiment performs control using a surface electro myogram/myoelectricity (EMG), which is a command signal from the upper center generated in the form of a potential on the muscle surface via the spinal cord, and further, in order to reduce a load on or a feeling of discomfort in muscles or joints, controls compensation through impedance adjustment that applies moment of inertia and viscoelasticity. The above-described biological signal measuring wearing device in the present invention is used in the EMG measurement.

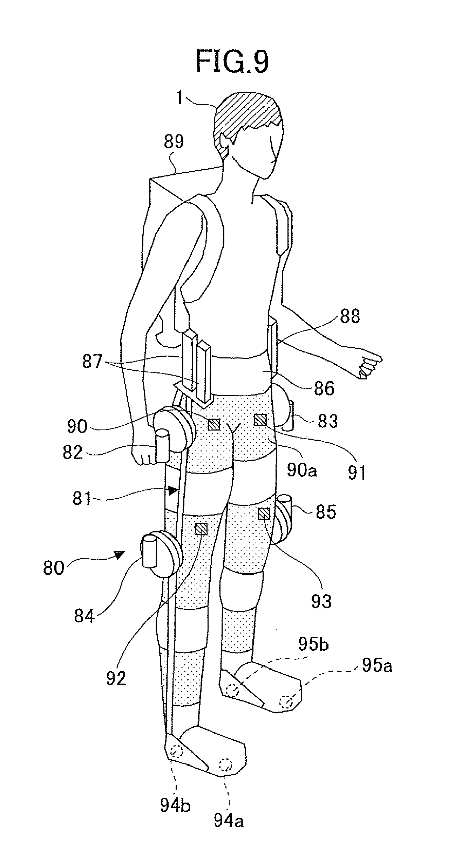

FIG. 9 is a diagram illustrating a wearable motion assisting apparatus to which the biological signal measuring wearing device is applied. FIG. 9 illustrates a case where a lower-body wearable motion assisting apparatus in this embodiment is worn. Further, in the case illustrated in FIG. 9, a wearable motion assisting apparatus 80 is an apparatus configured to assist the walking motion of, for example, a person physically challenged in the lower-limb motor function, having difficulty in walking because of reduction in the muscular strength of skeletal muscles or a person having difficulty in walking independently, such as a patient who goes through walking exercise rehabilitation. For example, these motions are made by detecting of a biological signal (for example, a signal generated from a human body such as a surface EMG) generated when a muscular force is generated in response to a signal from the brain, so as to provide a driving force from an actuator based on this detected signal.

Accordingly, the wearable motion assisting apparatus 80 illustrated in FIG. 9 is totally different from a so-called "playback robot," which is configured to have a robot hand computer-controlled based on preinput data, and is also called "powered suit," etc.

Further, the wearable motion assisting apparatus 80 illustrated in FIG. 9 is for the lower body, and the above-described biological signal measuring wearing device 10-2 is attached inside the wearable motion assisting apparatus 80. However, in the present invention, the wearable motion assisting apparatus 80 is not limited to this, and may be for the upper body, the whole body, or an individual part such as a hand, foot, finger, neck, etc., for example. That is, according to the present invention, a corresponding biological signal measuring wearing device is attached to the wearable motion assisting apparatus in accordance with its purpose of use.

Accordingly, the biological signal measuring wearing device transmits a biological signal obtained from the wearer 1 to the wearable motion assisting apparatus 80, and a driving part (an actuator) at a position to assist the motion of a part from which the biological signal has been obtained in the wearable motion assisting apparatus 80 illustrated below is driven based on the information.

When the wearer 1 wearing the wearable motion assisting apparatus 80 makes a voluntary walking motion, a driving torque corresponding to a biological signal generated at the time is given to the wearer 1 as an assisting force from the wearable motion assisting apparatus 80, so as to allow the wearer 1 to walk with half of the muscular force required for normal walking. Accordingly, the wearer 1 is enabled to walk while bearing her/his full weight with the resultant force of her-his own muscular force and the driving torque from the actuator (for which, for example, a power-operated drive motor is used in this embodiment).

At this point, as described below, the wearable motion assisting apparatus 80 performs control so that the assisting force (motor torque) given in accordance with a shift of the center of gravity caused by the walking motion reflects the intention of the wearer 1. Therefore, the actuator of the wearable motion assisting apparatus 80 is so controlled as to not impose a load that is against the intention of the wearer 1 and not hinder the motion of the wearer 1.

Further, in addition to a walking motion, the wearable motion assisting apparatus 80 may assist, for example, a motion at the time of the sitting wearer 1 standing up from a chair, a motion at the time of the standing wearer 1 sitting on a chair, a running motion, etc. Further, it is also possible to power-assist the wearer 1 when the wearer 1 goes up or down the stairs. In particular, when muscles are weak, it is difficult to make the motion of going up the stairs or the motion of standing up from a chair. However, the wearer 1 wearing the wearable motion assisting apparatus 80 is given a driving torque according to her/his own intention to be able to move without being bothered by reduction in muscular strength.

Here, a description is specifically given of each configuration of the wearable motion assisting apparatus 80 illustrated in FIG. 9. As illustrated in FIG. 9, the wearable motion assisting apparatus 80 includes a motion assisting wearing device 81 provided with actuators (corresponding to drive sources) to be worn by the wearer 1.

The actuators include, for example, a right thigh driving motor 82 positioned at the right hip joint of the wearer 1, a left thigh driving motor 83 positioned at the left hip joint of the wearer 1, a right knee driving motor 84 positioned at the right knee joint of the wearer 1, and a left knee driving motor 85 positioned at the left knee joint of the wearer 1. These driving motors 82 through 85, which are drive sources formed of servo motors whose driving torques are controlled with control signals from a controller, have respective speed reducing mechanisms configured to reduce the speed of motor rotation at a predetermined speed reducing ratio. The driving motors 82 through 85 are small in size but are capable of providing sufficient driving power.

Further, the wearable motion assisting apparatus 80 includes a waist belt 86 to be attached around the waist of the wearer 1. Batteries 87 and 88 to operate as a power supply for driving the driving motors 82 through 85 are attached to the waist belt 86. The batteries 87 and 88, which are rechargeable batteries, are disposed separately on the right side and the left side so as not to hinder a walking motion of the wearer 1.

Further, the wearable motion assisting apparatus 80 includes a control back 89 to be attached to the back of the wearer 1. For example, devices such as a control unit, a motor driver, a power supply circuit, and a display unit are housed in the control back 89. Further, the control back 89 is where the input/output part 68 of the above-described measuring device 13-2 is connected in the case of establishing an external connection. The above-described measuring device 13-2 may be housed inside the control back 89. The control back 89 is attached with its lower part supported by the waist belt 86, so that the weight of the control back 89 becomes no load on the wearer 1.

That is, according to the wearable motion assisting apparatus 80, for example, biological signals transmitted by wired or radio communications as described above from the biological signal measuring wearing device are obtained with the above-described measuring device 13-2 provided in the control back 89, and the control unit controls the operations of devices such as the motor driver and the power supply circuit based on the obtained biological signals.

Further, as the above-described electrode parts provided in the biological signal measuring wearing device according to the present invention, the wearable motion assisting apparatus 80 includes, for example, a bioelectric potential sensor (EMG sensor) 90 configured to detect a surface EMG caused by a motion of the right thigh of the wearer 1 (EMGhip), a bioelectric potential sensor (EMG sensor) 91 configured to detect a surface EMG caused by a motion of the left thigh of the wearer 1 (EMGhip), a bioelectric potential sensor (EMG sensor) 92 configured to detect a surface EMG caused by a motion of the right knee of the wearer 1 (EMGknee), and a bioelectric potential sensor (EMG sensor) 93 configured to detect a surface EMG caused by a motion of the left knee of the wearer 1 (EMGknee). In FIG. 9, bioelectric potential sensors are provided not only on the extensor side but also at corresponding positions on the flexor side of both thighs of the wearer 1. The bioelectric potential sensors 90 through 93 have respective pairs of electrode parts, and detect biological signals from a difference in potential between their respective paired electrode parts.

These bioelectric potential sensors 90 through 93, which are, for example, detecting parts configured to measure surface EMGs at the time of muscle power generation by skeletal muscles, include respective electrode parts configured to detect weak potential generated in skeletal muscles. In this embodiment, the bioelectric potential sensors 90 through 93 are provided on the inner surface of a leggings-like wearing device body 90a to be worn on the lower body by the wearer 1.

Here, a human body has multiple muscles from the waist below for moving the legs, such as the lliopsoas muscles for raising the thighs to the front side, the gluteus maximus muscle for lowering the thighs, the quadriceps femoris muscles for stretching the knees, and the biceps femoris muscles for flexing the knees. The bioelectric potential sensors 90 through 93 are provided at such positions in the vicinity of these muscles as to facilitate acquisition of bioelectric potential signals.

The bioelectric potential sensors 90 and 91 are so disposed as to come into contact with the anterior side of the groin areas of the wearer 1 with the wearing device body 90a being worn by the wearer 1, and measure EMGs corresponding to muscular forces at the time of bringing the legs forward by detecting the surface EMGs of the lliopsoas muscles. Further, the bioelectric potential sensors on the flexor side, present at the positions opposed to the positions of the bioelectric potential sensors 90 and 91, are so disposed as to come into contact with the buttocks of the wearer 1 with the wearing device body 90a being worn by the wearer 1, and measure, for example, EMGs corresponding to backward kicking forces or muscular forces at the time of going up the stairs by detecting the surface EMGs of the gluteus maximus muscle.

The bioelectric potential sensors 92 and 93 are so disposed as to come into contact with the upper anterior side of the knees of the wearer 1 with the wearing device body 90a being worn by the wearer 1, and measure EMGs corresponding to muscular forces to bring forward the legs from the knees below by detecting the surface EMGs of the quadriceps femoris muscles. Further, the bioelectric potential sensors 92 and 93 are so disposed as to come into contact with the lower posterior side of the knees of the wearer 1 with the wearing device body 90a being worn by the wearer 1, and measure EMGs corresponding to muscular forces to return backward the legs from the knees below by detecting the surface EMGs of the biceps femoris muscles. Accordingly, the wearable motion assisting apparatus 80 is configured so that driving currents to be supplied to the four driving motors 82 through 85 are determined based on the surface EMGs detected with these bioelectric potential sensors 90 through 93, and the driving motors 82 through 85 are driven with these driving currents, thereby giving an assisting force to assist a walking motion of the wearer 1.

Further, it is necessary to detect loads applied on the soles in order to smooth a walking motion. Therefore, reaction force sensors 94a, 94b, 95a, and 95b are provided on the right and left soles of the wearer 1 (indicated by broken lines in FIG. 9).

The reaction force sensor 94a detects a reaction force to a load on the anterior side of the right foot, and the reaction force sensor 94b detects a reaction force to a load on the posterior side of the right foot. The reaction force sensor 95a detects a reaction force to a load on the anterior side of the left foot, and the reaction force sensor 95b detects a reaction force to a load on the posterior side of the left foot. Each of the reaction sensors 94a, 94b, 95a, and 95b, which includes, for example, a piezoelectric element configured to output a voltage corresponding to an applied load, may detect a change in the load due to a shift of the body weight and the presence or absence of the contact of a foot of the wearer 1 with the ground.

### [Third Embodiment]

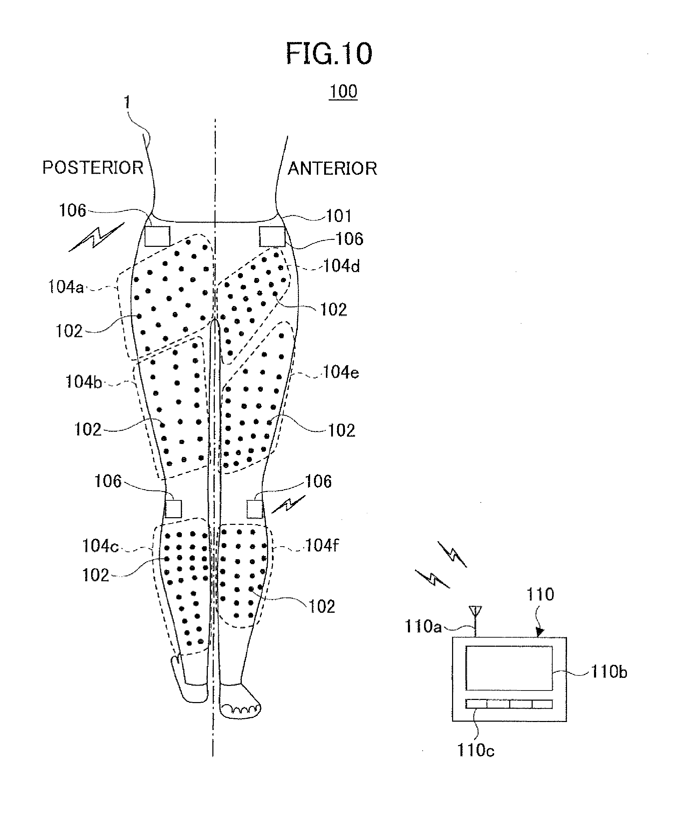

Further, a description is given below, using FIG. 10 through FIG. 13B, of an embodiment other than the above-described embodiments as a third embodiment. FIG. 10 is a schematic diagram illustrating the exterior of a biological signal measuring wearing device having biological signal sensor groups on a lower-body (legs) wearing device body. FIG. 10 is a schematic diagram illustrating, as an example of this embodiment, the exterior of a biological signal measuring wearing device 100 having a lower-body (legs) wearing device body 101 provided with biological signal sensor groups (biological signal detecting part groups) 104a through 104f including multiple biological signal sensors 102 (biological signal detecting parts). Further, the biological signal measuring wearing device 100 of this embodiment has a symmetrical structure. Thus, a structure on the posterior side of the left leg is illustrated in the left half of the drawing, and a structure on the anterior side of the left leg is illustrated in the right half of the drawing.

The wearing device body 101 has a pants-like (or leggings-like) shape so as to cover the wearer 1 from both legs up to the waist, and is formed of stretchable cloth. This wearing device body 101 is formed to be slightly smaller than the body size of the wearer 1. When the wearing device body 101 is worn by the wearer 1, the cloth stretches to allow the wearing device body 101 to change to a shape that matches the body shape of the wearer 1. Therefore, when the wearing device body 101 is worn by the wearer 1, the wearing device body 101 fits moderately to the body surface of the wearer 1 so as to allow the biological signal sensors 102 provided on the inner surface (a surface contacting the body surface of the wearer 1) to come into close contact with the skin surface of the wearer 1.

The wearing device body 101 is provided with the biological signal sensor groups 104a through 104f formed of the multiple biological signal sensors 102 configured to detect biological signals of the wearer 1. The biological signal sensors 102 are arranged at equal intervals along a flow of the leg muscles of the wearer 1. The biological signal sensors 102 are arranged along the flow of the gluteus maximus muscle in a part of the wearing device body 101 corresponding to the buttocks of the wearer 1 (the biological signal sensor group 104a). Likewise, the biological signal sensors 102 are arranged along a flow of the biceps femoris muscle, the semimembraneous muscle, and the semitendinosus muscle in a part of the wearing device body 101 corresponding to the posterior side of a thigh of the wearer 1 (the biological signal sensor group 104b), and are arranged along the flow of the triceps surae muscle in a part of the wearing device body 101 corresponding to a calf of the wearer 1 (the biological signal sensor group 104c). Further, the biological signal sensors 102 are arranged along a flow of the long adductor muscle and the iliopsoas muscles in a part of the wearing device body 101 corresponding to the anterior side of a hip joint (the biological signal sensor group 104d), are arranged along the flow of the quadriceps femoris muscle in a part corresponding to the anterior side of a thigh (the biological signal sensor group 104e), and are arranged along a flow of the anterior tibial muscle, the soleus muscle, and the extensor digitorum longus muscle in a part of the wearing device body 101 corresponding to a shin (the biological signal sensor group 104f).

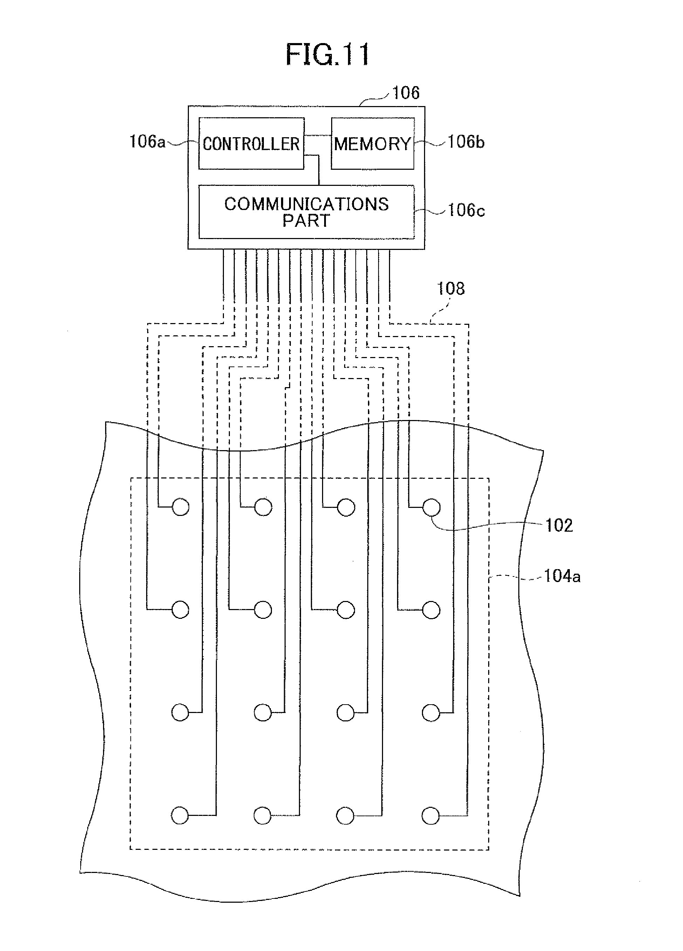

Here, FIG. 11 is a diagram illustrating a measuring module (a biological signal obtaining part) connected to a biological signal sensor group provided on a wearing device body. The biological signal sensor groups 104a through 104f provided on the wearing device body 101 are connected to respective measuring modules in the same manner. Accordingly, a description is given herein, taking the biological signal sensor group 104a as an example.

FIG. 11 is a schematic diagram illustrating the biological signal sensor group 104a provided on the wearing device body 101 and a measuring module 106 (a biological signal obtaining part) connected to this biological signal sensor group 104a. The individual biological signal sensors 102 of the biological signal sensor group 104a are provided in electrical isolation from one another, and are connected to the measuring module 106 via respective electrically conductive interconnects 108. These biological signal sensors 102 are assigned respective addresses.

This measuring module 106 includes a flat integrated circuit (IC), and is so positioned as to be less likely to hinder a motion of the wearer 1, for example, around the waist or a shin of the wearing device body 101 as illustrated in FIG. 10, and is fixed to the wearing device body 101. The measuring module 106 may be, for example, housed in a pocket provided on the wearing device body 101, detachably fixed using a hook-and-loop fastener or the like, or undetachably fixed by sewing or the like.

The measuring module 106 includes a measuring module controller 106a connected to the biological signal sensors 102 of the biological signal sensor group 104a and configured to select at least two from these biological signal sensors 102 and obtain a biological signal by determining a difference between detection signals detected with these selected biological signal sensors 102; a memory 106b in which the obtained biological signal is recorded; and a communications part 106c configured to transmit successively obtained biological signals and/or biological signals recorded in the memory 106b to the outside. The biological signal sensor groups 104a through 104f are provided with their respective measuring modules 106, which are connected to the respective biological signal sensors 102.

The measuring module controller 106a includes an electronic circuit capable of successively selecting at least two of the connected biological signal sensors 102 in response to a command signal input via the communications part 106c and obtaining a biological signal between these selected two biological signal sensors 102. In addition, the measuring module controller 106a further includes signal processing parts such as a filter configured to remove or extract a predetermined frequency component from the biological signal thus obtained and an amplifier configured to amplify the obtained biological signal. The biological signal thus obtained is output from the measuring module controller 106a to the communications part 106c and/or the memory 106b. In making selections from the biological signal sensors 102, the measuring module controller 106a may be configured to successively operate the biological signal sensors 102 in preset order or to select, based on a command signal input via the communications part 106c, the biological signal sensors 102 of the addresses specified by the command signal.

The communications part 106c includes a flat antenna and a communications circuit connected to this antenna. The communications part 106c transmits, to a below-described measuring unit via the antenna, measurement information (measurement data) containing a signal including a biological signal output from the controller 106a and information such as address information indicating the position information of the biological signal sensors 102 that have detected the biological signal and/or a signal including a biological signal read from the memory 106b and information such as address information indicating the position information of the biological signal sensors 102 that have detected the biological signal.

A measuring unit 110 illustrated in FIG. 10, which has a reader/writer function to transmit a signal of the start or end of data obtaining and a data storing function, includes an antenna 110a configured to receive a signal output from the measuring module 106 and to transmit a signal to the measuring module 106; a memory configured to record biological signals measured by the biological signal sensor groups 104a through 104f; and a controller configured to generate a command signal to the measuring module 106 and control communications with an external apparatus. In addition, the measuring unit 110 may further include an input part 110c including a keyboard and operating buttons and a display part 110b to display measured data, such as a monitor.

The controller of the measuring unit 110 generates a specifying signal to specify the biological signal sensors 102 to be selected from each of the biological signal sensor groups 104a through 104f provided on the wearing device body 101 and a signal of the start or end of data obtaining, and transmits these signals to the measuring module 106 via the antenna 110a. The measuring module 106 selects the specified biological signal sensors 102 and measures a biological signal in accordance with the received signals.

Further, in the case of further wearing the wearable motion assisting apparatus 80 (see FIG. 9) over the wearing device body 101 of the biological signal measuring wearing device 100 according to the present invention, the measuring unit 110 is preferably housed in the wearable motion assisting apparatus 80. In this case, the measuring unit-side antenna 110a is preferably provided near the antenna of the communications part 106c of the measuring module 106 of the wearing device body 101, particularly at a position opposed to the antenna of the communications part 106c, with the biological signal measuring wearing device 100 and the wearable motion assisting apparatus 80 being worn by the wearer 1. Further, in place of such radio communications via an antenna, signals may be transmitted and received by wired communications performed via connectors.

Thus, according to the biological signal measuring wearing device 100 of this embodiment, the wearing device body 101 is provided with the biological signal sensor groups 104a through 104f formed of the multiple biological signal sensors 102. Therefore, wearing the wearing device body 101 alone makes it possible to place the multiple biological signal sensors 102 in predetermined regions at a time and to detect biological signals at respective points with the biological signal sensors 102 in close contact with the skin surface. Thus, also in the case of monitoring biological signals at multiple points on the body surface of the wearer 1, it is possible to save the trouble of attaching or detaching the biological signal sensors 102 one by one, thus making it possible to measure biological signals with ease.

Further, by thus detecting biological signals with the multiple biological signal sensors 102, it is possible to measure a biological signal at each of multiple points in the regions where the biological signal sensor groups are placed. By mapping measurement data at each point in accordance with the addresses assigned to the individual biological signal sensors 102, it is possible to measure a biological signal distribution in the body of the wearer 1.

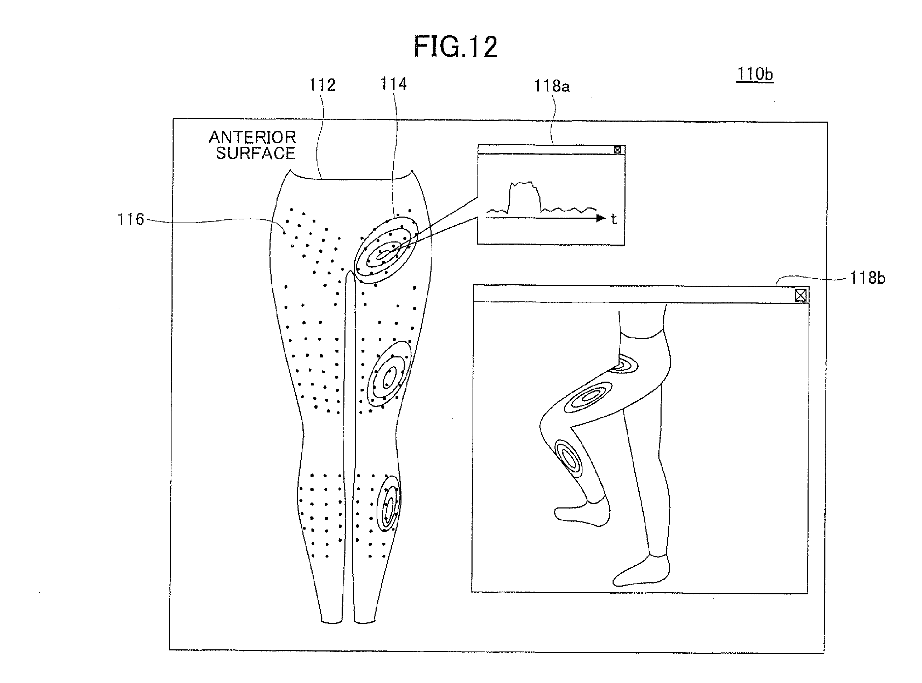

Here, FIG. 12 is a diagram illustrating a biological signal distribution using the biological signal measuring wearing device. FIG. 12 illustrates a case where a biological signal distribution is displayed via the display part 110b provided in the measuring unit 110 as illustrated in FIG. 10, using the biological signal measuring wearing device 100.

A model 112 showing a general contour of the wearing device body 101 is displayed on the monitor (display part) 110b provided in the measuring unit 110. The distribution of the biological signals measured with the biological sensor unit groups 104a through 104f is displayed as contour lines 114 on this model 112. Displaying the biological signal distribution with different colors makes it easier to visually recognize the biological signal distribution. Further, pointers 116 may be displayed in correspondence to the displayed positions of the biological signal sensors 102, and a desired position may be selected from among the pointers 116 to display the waveform of a biological signal with passage of time t of the selected pointer 116 in a window 118a.

Further, as illustrated in another window 118b, an actual motion of the wearer 1 may be captured with a camera or the like, and the data of the measured biological signals may be displayed, being mapped onto the video in a superposed manner. At this point, providing pointers to serve as marks on the front side (outer side) of the wearing device body 101 at positions corresponding to the biological signal sensors 102 allows the positions of the pointers to be recognized as they are as the positions of the biological signal sensors 102 in the captured video of the motion of the wearer 1, thus making it possible to perform mapping with efficiency. For example, stickers or patches different in color from the wearing device body 101 may be applied as pointers. The contents of display of the above-described windows 118a and 118b and the size, positions, number, etc., of windows are not limited in particular in the present invention.

Further, application of biological signals measured with this biological signal measuring wearing device 100 to the control of the wearable motion assisting apparatus 80 has the following advantages. Since the wearing device body 101 is provided with the multiple biological signal sensors 102, it is possible to attach the multiple biological signal sensors 102 together by putting on the wearing device body 101. This eliminates the necessity of putting on the biological signal sensors 102 by, for example, attaching them one by one, thus making it easy to attach the biological signal sensors 102. Further, since the biological signal sensors 102 are fixed to the wearing device body 101, it is possible to keep the biological signal sensors 102 at regular intervals, thus making it possible to measure biological signals under uniform conditions.

Further, since it is possible to measure biological signals at multiple points, it is possible to select points where biological signals are detected with relative ease from among the multiple points and therefore to obtain signals suitable for controlling the wearable motion assisting apparatus 80 even if there is difficulty in detecting biological signals for controlling the wearable motion assisting apparatus 80 for some reason such as the weakening of biological signals due to a disordered body function. Accordingly, it is easy to measure biological signals necessary for controlling the wearable motion assisting apparatus 80.

Displaying images or the like on the display part 110b as illustrated in FIG. 12 is executed with predetermined application software, and the contents of the execution and the contents of display on the display part 110b may be recorded in a recording part such as a hard disk.

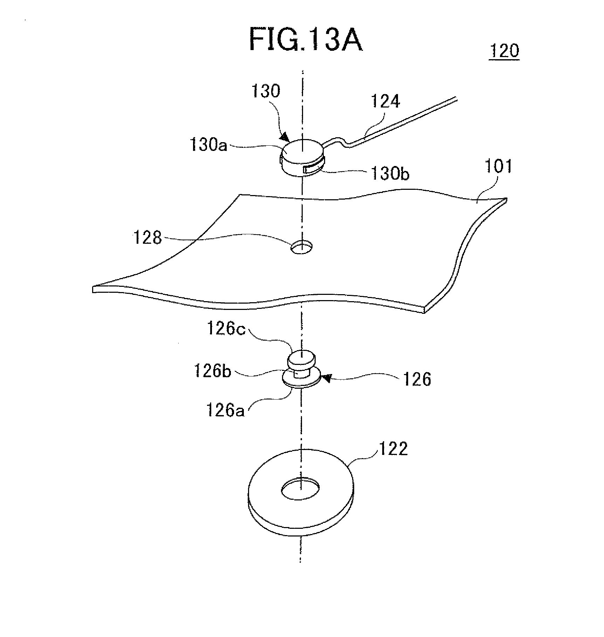

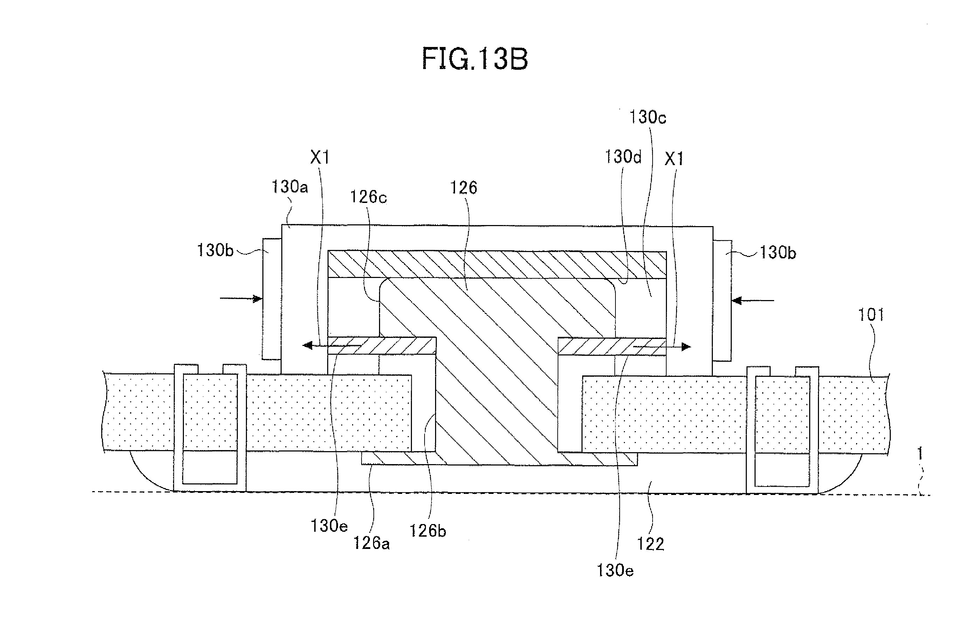

Next, a description is given of a specific structure of each of the biological signal sensors 102. FIG. 13A is an exploded perspective view illustrating an outline of the configuration of the biological signal sensor 102. Further, FIG. 13B is a schematic enlarged view of a cross section of the biological signal sensor 102 in an assembled state. In the following description, for convenience, the upper side in the drawings (the outer side of the wearing device body 101) may be referred to as "upper side," and the lower side in the drawings (the inner side of the wearing device body 101, that is, the side to contact the skin surface of the wearer 1) may be referred to as "lower side."

As illustrated in FIGS. 13A and 13B, the biological signal sensor 102 has a configuration where an electrode part 120 configured to come into contact with the skin surface of the wearer 1 to detect a biological signal is formed unitarily with the wearing device body 101. This electrode part 120 includes a thin disk-shaped electrode pad 122 formed of, for example, an electrically conductive polymer gel or the like and a connecting terminal 126 electrically connecting this electrode pad 122 and an interconnect 124.

The electrode pad 122 has such flexibility and adhesiveness as to deform to adhere to the skin surface of the wearer 1 when coming into contact with the skin surface. This allows the electrode pad 122 to come into close contact with the skin surface of the wearer 1 and establish a good electrical connection.

Further, the connecting terminal 126, electrically connecting the electrode pad 122 and the interconnect 124 (corresponding to the interconnect 108 connecting the biological signal sensor 102 and the measuring module 106 illustrated in FIG. 11), is formed of an electrically conductive metal, and includes a disk-shaped fixing part 126a smaller than the electrode pad 122 and a substantially pillar-shaped terminal part 126b formed to project from the upper surface of the fixing part 126a. A connecting projection part 126c formed to bulge outward relative to the circumferential surface of this columnar terminal part 126b is provided at the end of the terminal part 126b. Further, the wearing device body 101 is provided with an opening part 128 through which the terminal part 126b of the connecting terminal 126 is insertable.

The connecting terminal 126 is fixed unitarily (in advance) to the center portion of the electrode pad 122, so that the electrode pad 122 is positioned relative to the wearing device body 101 by the insertion of the terminal part 126b of the connecting terminal 126 through the opening part 128 of the wearing device body 101.

Further, the fixation of the electrode pad 122 to the wearing device body 101 prevents the electrode pad 122 from being displaced or turned up and allows a good electrical connection to be established between the electrode pad 122 and the skin surface of the wearer 1 in cases such as when the wearer 1 puts on or takes off the wearing device body 101 and when the wearer 1 makes a motion while wearing the biological signal measuring wearing device 100 and measuring biological signals.

At least one surface (a surface to come into contact with the skin of the wearer 1) of the electrode pad 122 is adhesive so as to allow close contact with the skin surface of the wearer 1 to have a good electrical connection. Providing the other surface of the electrode pad 122 with adhesiveness is effective in fixing the electrode pad 122 to the wearing device body 101.

Further, as illustrated in FIGS. 13A and 13B, in order to allow the electrode pad 122 and the interconnect 124 to be electrically connected with ease, the electrode pad 122 may be fixed to the wearing device body 101 using, for example, a clip connector 130 or the like at the end of the interconnect 124 so that the connector 130 fits to the connecting projection part 126c. In this case, the interconnect 124 and the connecting projection part 126c are allowed to be electrically connected at the time of fitting. It is preferable that the connector 130 be insulative.

In this embodiment, the terminal part 126b is inserted through the opening part 128 of the wearing device body 101, and with the electrode pad 122 being fixed to the wearing device body 101, the connector 130 is caused to fit to the connecting projection part 126c projecting from the opening part 128 so that the connecting projection part 126c connects to the interconnect 124. Here, the connector 130 is provided at the end of the interconnect 124, and has a pair of operations parts 130b provided at the side surface of a housing 130a.

Here, as illustrated in FIG. 13B, a housing hole 130c into which the connecting projection part 126c is fit and housed is formed inside the housing 130a having, for example, a substantially cylindrical shape, and the inner surface of the housing hole 130c is provided with an electrically conductive connecting surface 130d.

The connecting surface 130d is electrically connected to the interconnect 124 inside the housing 130a. Further, a connection piece 130e so provided at the entrance of the housing hole 130c as to project from the inner surface of the housing hole 130c is configured to retreat or project in the radial directions of the housing hole 130c in response to pressing or releasing the operations parts 130b. Accordingly, pressing the operations parts 130b provided on the side surface of the housing 130a causes the connection piece 130e to move in a direction to be housed inside the housing 130a (in the direction indicated by arrow X1 in FIG. 13B), so as to widen the entrance of the housing hole 130c. Housing the connecting terminal 126 inside the housing hole 130c and releasing the operations parts 130b in this state causes the connection piece 130e to move toward inside the housing hole 130c from the housing 130a, so that the end of the connection piece 130e returns to a position to contact the side surface of the terminal part 126b to narrow the entrance of the housing hole 130c. As a result, the connecting projection part 126c of the connecting terminal 126 runs into the connection piece 130e to prevent the terminal part 126b from coming out of the housing hole 130c, so that the connector 130c is fixed to the connecting terminal 126.

As illustrated in FIGS. 13A and 13B, connecting the electrode pad 122, which is the biological signal sensor 102, using the connector 130 allows the biological signal sensor 102 to be detachably fixed to the wearing device body 101. Further, fixing the measurement module 106 as well to the wearing device body 101 in a detachable manner (for example, using a hook-and-loop fastener or the like), for example, allows the wearing device body 101 to be kept clean because the whole wearing device body 101 is washable by removing electrical components such as the biological signal sensors 102 at the time of washing the wearing device body 101.

Thus, according to the above-described biological signal measuring wearing devices, it is possible to attach multiple biological signal detecting parts at a time by putting on the wearing device body, thus eliminating the necessity of putting on the biological signal detecting parts (biological signal sensors or bioelectric potential sensors) by, for example, attaching them one by one and thus facilitating attachment of the biological signal detecting parts at the time of detecting biological signals. Further, an increase in the number of biological signal detecting parts is inevitable in cases such as when measuring biological signals related to, for example, the motions of both arms, both legs, etc. According to the present invention, however, even in such cases, it is possible to attach multiple biological signal detecting parts together by putting on the wearing device body, thus facilitating attachment of the biological signal detecting parts. Further, since the biological signal detecting parts are fixed to the wearing device body, it is possible to keep the biological signal detecting parts at regular intervals, thus making it possible to measure biological signals under uniform conditions. Further, causing the wearable motion assisting apparatus 80 to operate using the biological signals makes it possible to provide highly accurate motion assistance. The above-described biological signal measuring wearing devices and wearable motion assisting apparatus may be applied to not only human bodies but also to animals and other living things (including plants).

As described above, according to the present invention, it is possible to provide a wearing signal measuring wearing device for facilitating attachment of biological signal sensors for obtaining biological signals from human bodies. Further, application of the biological signal measuring wearing device to, for example, the wearable motion assisting apparatus as illustrated in FIG. 9 makes it possible to reduce the trouble of directly attaching biological signal sensors such as electrode parts to or detaching them from predetermined positions on the wearer. Further, since it becomes easy to put on the wearable motion assisting apparatus, it is possible to reduce a load on the wearer.

That is, according to the present invention, biological signal sensors configured to detect biological signals for driving actuators of the wearable motion assisting apparatus are placed at positions where the sensors are to be attached (such positions as to facilitate acquisition of biological signals), for example, on the inner surface (a surface on the side to come into close contact with the body) of an inner suit. Accordingly, putting on this suit by a wearer makes it possible to detect biological signals in such a manner as if the sensors are stuck to predetermined positions on the skin surface of the wearer.

For example, the suit may be provided with interconnects with a connector to connect to the wearable motion assisting apparatus, and biological signals from the sensors may be transmitted to the wearable motion assisting apparatus via the interconnects in a wired manner. Alternatively, in the case where the electrode parts are provided with their respective small-size signal transmitting parts, the biological signals may be transmitted to the wearable motion assisting apparatus by radio.

A description is given above of preferred embodiments of the present invention. The present invention, however, is not limited to the specific embodiments, and variations and modifications may be made within the gist of the present invention described in CLAIMS.

The present international application is based on and claims the benefit of priority of Japanese Patent Application No. 2008-232091, filed on September 10, 2008, the entire contents of which are incorporated herein by reference.

### DESCRIPTION OF THE REFERENCE NUMERALS

1 wearer
10 biological signal measuring wearing device
11, 30 wearing device body
12, 24 electrode part
13 measuring device
20 glove
21 sock
22, 50 supporter
31 opening part
32 net member
33 electrode pad
34 cover member
35 mark part
36 attachment part
37 radio tag (RFID) part
40 skin (body surface)
41 rising portion
51 attachment part
60 display part
61 biological signal sensor
62 filter
63, 67, 71 controller
64, 72 memory
65, 66, 73, 74 communications part
68 input/output part
70 sensor unit
80 wearable motion assisting apparatus
81 motion assisting wearing device
82 right thigh driving motor
83 left thigh driving motor
84 right knee driving motor
85 left knee driving motor
86 waist belt
87, 88 battery
89 control back
90-93 bioelectric potential sensor
94, 95 reaction force sensor
100 biological signal measuring wearing device
101 wearing device body
102 biological signal sensor
104a-104f biological signal sensor group
106 measuring module
106a controller
106b memory
106c communications part
108 interconnect
110 measuring unit
110a antenna
110b display part
110c input part
112 model
114 contour line
116 pointer
118 window
120 electrode part
122 electrode pad
124 interconnect
126 connecting terminal
126a fixing part
126b terminal part
126c connecting projection part
128 opening part
130 connector
130a housing
130b operations part
130c housing hole
130d connecting surface
130e connection piece

## Claims

1. A biological signal measuring wearing device configured to measure a biological signal from a body surface of a wearer, the biological signal measuring wearing device comprising:
a wearing device body configured to cover the body surface of the wearer;
a biological signal detecting part provided on a predetermined part of an inner surface of the wearing device body and configured to detect the biological signal from the body surface of the wearer; and
a signal communicating part configured to output the signal detected by the biological signal detecting part.

2. The biological signal measuring wearing device as claimed in claim 1, comprising:
a biological signal obtaining part configured to obtain the biological signal from the signal detected by the biological signal detecting part.

3. The biological signal measuring wearing device as claimed in claim 2, comprising:
a plurality of the biological signal detecting parts,
wherein the biological signal obtaining part is provided for each of the individual biological signal detecting parts or for each of biological signal detecting part groups each of a preset number of the biological signal detecting parts.

4. The biological signal measuring wearing device as claimed in any of claims 1 to 3, comprising:
a plurality of the biological signal detecting parts,
wherein the signal communicating part is provided for each of the individual biological signal detecting parts or for each of biological signal detecting part groups each of a preset number of the biological signal detecting parts.

5. The biological signal measuring wearing device as claimed in any of claims 1 to 4, wherein:
the wearing device body is formed of a stretchable material to come into contact with the body surface and has an opening part provided in at least a part thereof,
the biological signal detecting part is placed so as to allow the biological signal from the body surface to be detected via the opening part, and
the signal communicating part is configured to transmit the biological signal detected by the biological signal detecting part by a wired or radio communication.

6. The biological signal measuring wearing device as claimed in claim 5, comprising:
a net member woven from a stretchable material provided between the opening part and the biological signal detecting part.

7. The biological signal measuring wearing device as claimed in claim 6, wherein the biological signal obtaining part is configured to obtain the biological signal by causing a portion thereof rising through mesh openings of the woven net member to come into close contact with the body surface of the wearer.

8. The biological signal measuring wearing device as claimed in claim 6 or 7, wherein the attachment part includes a mark part configured to allow the biological signal detecting part or the net member to be fixed at a predetermined position.

9. The biological signal measuring wearing device as claimed in any of claims 1 to 8, wherein the wearing device body is an inner suit, a legging, a supporter, a glove, or a sock.

10. A wearable motion assisting apparatus configured to control driving of a motion assisting wearing device having a drive source configured to provide a wearer with power, and to assist a motion of the wearer or make the motion for the wearer, based on a biological signal obtained from the biological signal measuring wearing device as set forth in any of claims 1 to 9.
